# EUROPEAN PATENT SPECIFICATION

(11) **EP 1 633 424 B1**
(45) Date of publication and mention of the grant of the patent: **24.10.2007**
(21) Application number: 03735993.2
(22) Date of filing: 16.06.2003
(51) Int. Cl.: A61M 25/10

(54) **Diagnostic catheter**
Diagnosekatheter
Catheter diagnostique

(43) Date of publication of application: 15.03.2006
(73) Proprietor: Artech S.r.l., 41032 Cavezzo (IT); ZATTERA, Giuseppe, 10100 Torino (IT); SORO, Elisabetta, 10100 Torino (IT)
(72) Inventor: ZATTERA, Giuseppe, I-10100 Torino (IT)
(74) Representative: Crippa, Paolo Ernesto
(86) International application number: PCT/IT2003/000374
(87) International publication number: WO 2004/110543

(56) References cited:
- US-A- 5 454 788
- US-A- 5 908 407
- US-A- 6 017 323
- US-B1- 6 558 401

## Description

The present invention relates to a catheter for medical use.

In particular, the present invention relates to a diagnostic catheter suitable for perfusing a substance, such as for example a contrast liquid, in blood vessels. A catheter according to the preamble of claim 1 is disclosed in US-A-5 908 407.

In the medical field, in particular in cardiac surgery, coronary by-pass intervention is very widespread, consisting, as the name indicates, in constructing "bridges" capable of passing over the coronary stenoses responsible for cardiac ischemia. As ducts for the by-passes, the internal mammary arteries (dx and sx) are by far preferred by surgeons for the greater viability of the intervention over the years. The right mammary artery is often anastomosed to the left in a "Y" so as to be able to reach with it all the vessels of the posterior surface of the heart. In order to be able to carry out such interventions it would be ideal to be able to verify in advance the calibre, length and viability of the vessels to be anastomosed. Catheters are known in the art which are suitable for infusing a contrast liquid into a vessel in such a way as to verify its anatomical characteristics (calibre and length) in advance.

Such techniques, defined as direct selective infusion, consist in directly selecting the vessel to be infused, by means of a catheter. They therefore involve the need to span with the catheter a main vessel from which branches off the vessel to be infused, and to enter the bifurcation with the catheter in order to be able to inject the substance directly into the preselected vessel.

These known catheters have extremely soft and flexible ends which have the task of adapting to the curvatures of the vessels to enter them.

Sometimes these catheters have the drawback of dissecting the vessels, in particular vessels of reduced calibre or having accentuated curvatures at the branchings from which they start. The lesions caused by incorrect insertion of a catheter may be extremely serious, so that such a known technique is not used for vessels having the above-mentioned characteristics.

It is known from US 5908407 and US 6558401 B1, to provide catheters comprising occluding means both upstream and downstream an aperture to deliver a fluid in a selected vein. These known catheters are not able to perfuse small quantities of a liquid directly in a target vein.

The problem underlying the present invention is that of providing a catheter which solves the drawbacks cited with reference to the prior art.

Such drawbacks and limitations are solved effectively by a catheter according to claim 1.

Other embodiments of the catheter according to the invention are described in the subsequent claims.

Further characteristics and advantages of the invention in question will become clearer from the following description of some of its preferred and nonlimiting exemplary embodiments, in which:
Figure 1 shows a side view in section of a catheter according to one embodiment of the invention, in a non-operative state;
Figure 2 shows a side view in section of the catheter of Figure 1 in an operative state;
Figure 3 shows a side view in section of the catheter of Figure 1, inserted into a vessel and in an operative state;
Figures 4A-4D show side views of occluding bodies according to different embodiments of the invention;
Figure 5 shows a sectional view of a catheter according to a further embodiment of the invention;
Figure 6 shows a sectional view of a catheter according to a further embodiment of the invention;
Figure 7 shows a sectional view of the catheter of Figure 1 along the line VII-VII of Figure 1;

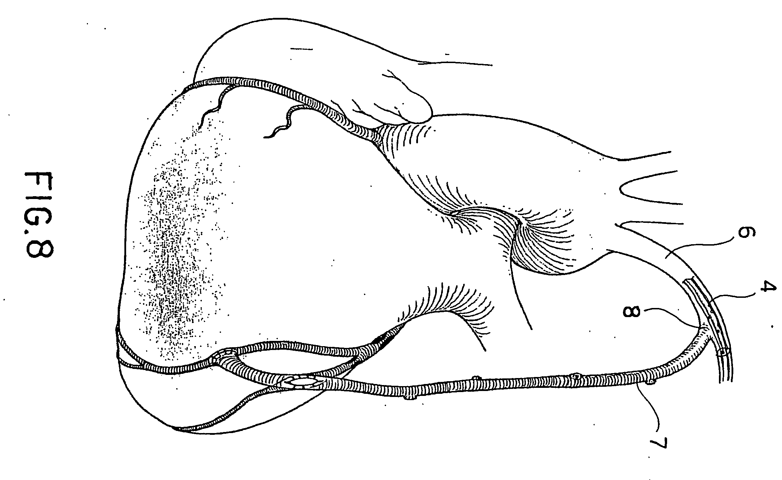

Figure 8 shows a diagrammatic view illustrating an application of said catheter within a subclavian artery for infusing a liquid in a mammary artery; and

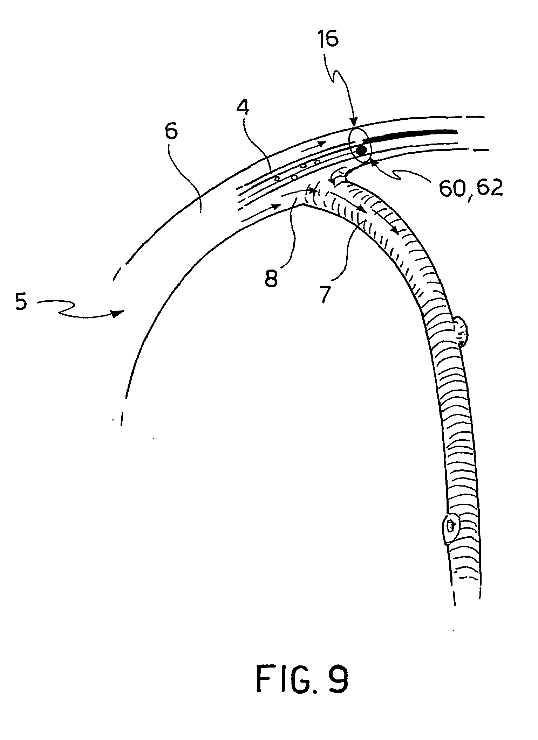

Figure 9 shows an enlarged detail of Figure 8.

Elements or parts of elements common to the embodiments described hereinafter will be indicated by the same numerical references.

With reference to the aforesaid figures, the reference 4 indicates generally a catheter for medical use according to the invention, suitable for being inserted into a duct 5 comprising a first blood vessel 6, for example an artery, and a second blood vessel 7 which branches off from the first vessel 6 at a bifurcation 8, in order to perfuse a substance from the first vessel 6 to the second vessel 7, without cannulating the second vessel 7.

By the term first vessel there is to be understood the vessel cannulated directly by the catheter 4 and from which starts or branches off the second vessel 7, in which it is desired to infuse a substance.

This definition makes no reference to the function or to the characteristics which said vessels have within the circulatory system, so that the first vessel 6 is the vessel to be cannulated and the second vessel 7 is the vessel to be perfused indirectly, or without direct insertion of the catheter 4.

The catheter 4 comprises a catheter body 10, having a substantially tubular shape, which extends from a proximal end 12 to a distal end 16, along a main axis of extension X.

The catheter body 10 comprises a main cavity 20 which passes through it between the proximal end 12 and the distal end 16 and preferably having a circular cross-section.

The main cavity 20 is suitable for receiving a guide cable, not shown, for the insertion of the catheter 4.

In an intermediate position between the proximal end 12 and the distal end 16, preferably in proximity to the distal end 16, the catheter body 10 comprises at least one opening 24, preferably a plurality of openings, suitable for perfusing a substance and passing through a lateral wall 28 of the catheter body 10, so as to place the main cavity 20 in fluid communication with the surroundings into which the catheter 4 is being introduced, for example with the cavity of the first vessel 6 bounded by an inner wall 32 of said first vessel 6.

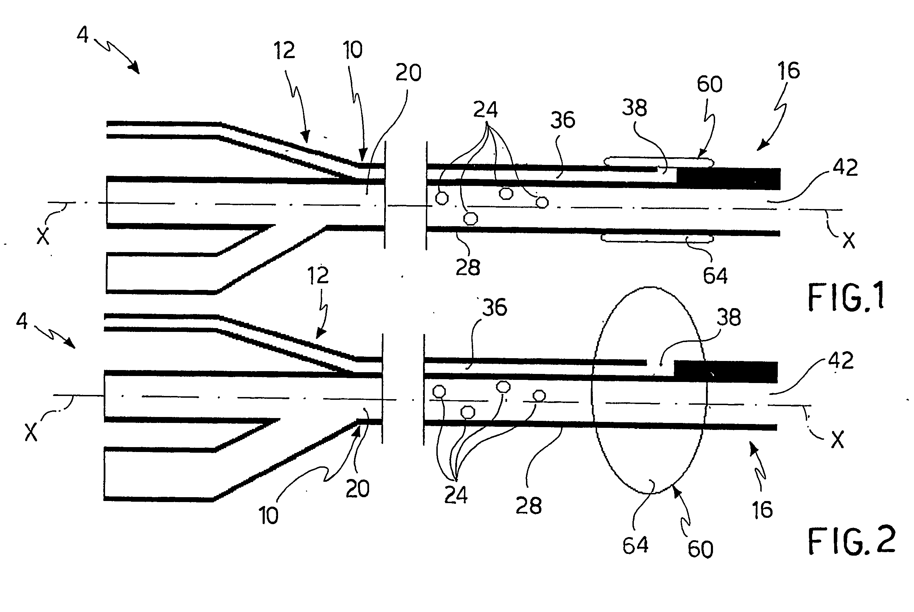

The openings 24, as shown for example in Figures 1-3, are not aligned with one another but are preferably disposed in a helical direction with respect to the axis X.

Advantageously, said openings 24 are of such dimensions that the sum of the areas of the openings 24 is not less than the area of the main cavity 20 of the catheter body 10 at the distal end 16.

According to a preferred embodiment, the catheter body 10 comprises a secondary cavity 36 which passes through the catheter body 10 from the proximal end 12 to the distal end 16, extending, for example, parallel to the main cavity 20.

The secondary cavity 36 is fluidly separated from the main cavity 20, or the two cavities, main and secondary 20, 36, are not in fluid communication with each other.

Preferably, the second cavity 36 is provided in a thickness of the lateral wall 28 of the catheter body 10.

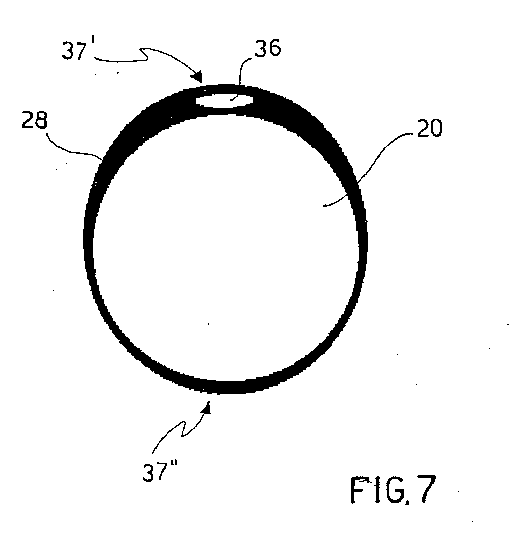

As emphasized in Figure 7, the catheter body 10 preferably has an oval, or substantially elliptical cross-section, having a first pole 37' more pronounced than a second pole 37" diametrically opposed to the first pole 37'. At the first pole 37', the thickness of the lateral wall 28 of the catheter body is slightly greater so as to receive the secondary cavity 36.

Preferably, the secondary cavity 36, at the distal end 16, opens externally of the catheter body 10; for example, it opens into a lateral hole 38 located on the lateral wall 28 of the body itself.

Preferably, the lateral hole 38 is positioned along the catheter body 10 between the plurality of openings 24 and the distal end 16.

The catheter body 10, at the distal end 16, comprises a main aperture 42 into which opens the main cavity 20 having, according to one embodiment, a calibre substantially similar to the median calibre of the catheter body 10.

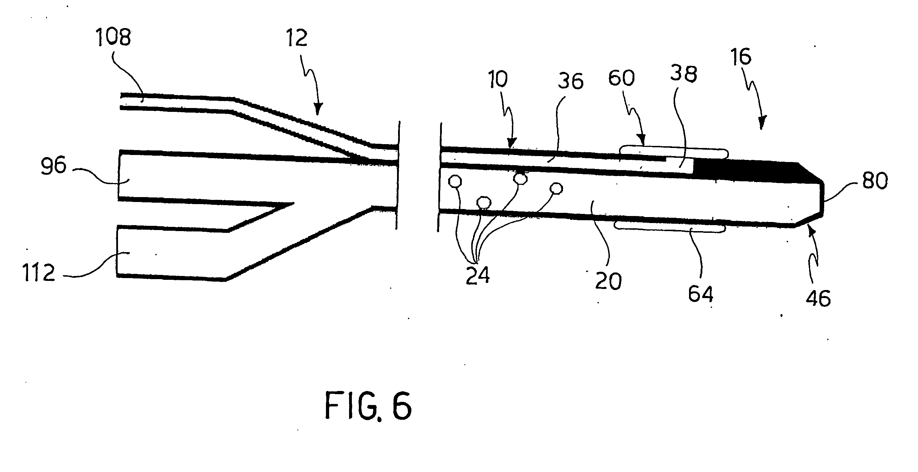

According to one embodiment, illustrated for example in Figure 6, the catheter body 10, at the distal end 16, has a portion with tapered profile 46, for example a frustoconical profile, such that the calibre of the main aperture 42 is less than the median calibre of the catheter body 10.

The calibre of the main aperture 42 is however such as to allow the passage of a guide cable for the insertion of the catheter 4 into a vessel.

The catheter 4 comprises first occluding means 60 and second occluding means 62, in which the first occluding means 60 are suitable for at least partially occluding a gap 63 between the catheter body 10 and the first vessel 6 into which the catheter 4 is inserted, and the second occluding means 62 can be associated internally with the main cavity 20 and suitable for at least partially occluding said main cavity 20 of the catheter body 10.

The first and second occluding means 60, 62 define a preferred direction of outflow of a fluid from the main cavity 20 of the catheter body 10 through the openings 24 and, advantageously, from the openings 24 to the second vessel 7 which branches off from the first vessel 6.

In other words, the first and second occluding means 60, 62 co-operate with one another to create a resistance to the passage of fluid beyond the distal end, favouring the outflow of fluid through said openings 24, so as to perfuse the fluid indirectly into the second vessel 7, avoiding the dispersion of fluid in the first vessel 6.

In yet other words, the first and second occluding means 60, 62, at a portion of the catheter body 10 comprised between the openings 24 and the distal end 16, substantially effect the occlusion of the cavity of the first vessel 6 into which the catheter 4 is inserted so as to oppose a flow of fluid in the direction of the axis X through the distal end 16 and direct a flow of fluid from the openings 24 of the catheter body 10 towards the second vessel 7.

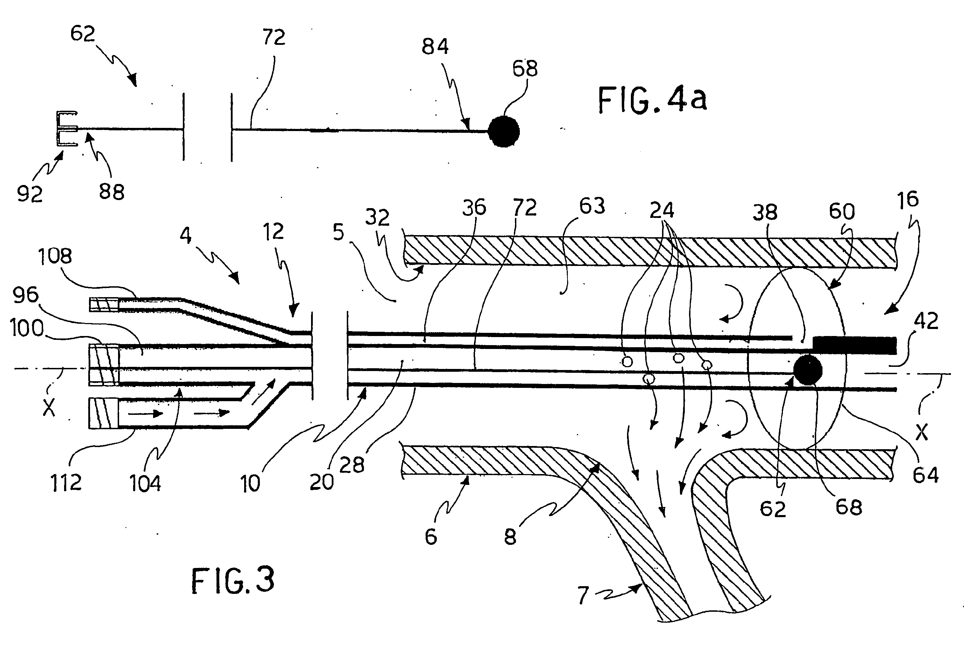

The first occluding means 60, as illustrated for example in Figure 3, comprise an inflatable element 64, positioned round the lateral wall 28 of the catheter body 10 and advantageously in fluid connection with the lateral hole 38 of the secondary cavity 36, so as to be able to be actuated from the proximal end 12 through the secondary cavity 36.

The inflatable element 64 in a non-operative state, illustrated for example in Figure 1, adheres substantially to the lateral wall 28 of the catheter body 10, while in an operative or working state, illustrated for example in Figure 2, in which it fills with a substance injected for example through the secondary cavity 36, the inflatable element 64 expands so as to be substantially in contact with the inner wall 32 of the first vessel 6. In other words, the inflatable element 64, in a working state, tends to occupy the substantially toroidal gap 63 comprised between the lateral wall 28 of the catheter body 10 and the inner wall 32 of the first vessel 6 into which the catheter is inserted.

The second occluding means 62 comprise an occluding body 68, suitable for being introduced into the main cavity 20, and an insertion cable 72 for insertion of the occluding body 68, firmly connected to the occluding body 68, suitable for allowing the insertion and positioning of the occluding body 68 into the main cavity 20.

According to one embodiment, shown for example in Figure 4A, the occluding body 68 is substantially spherical in shape, such that a diameter of the occluding body is smaller than the calibre of the main cavity 20.

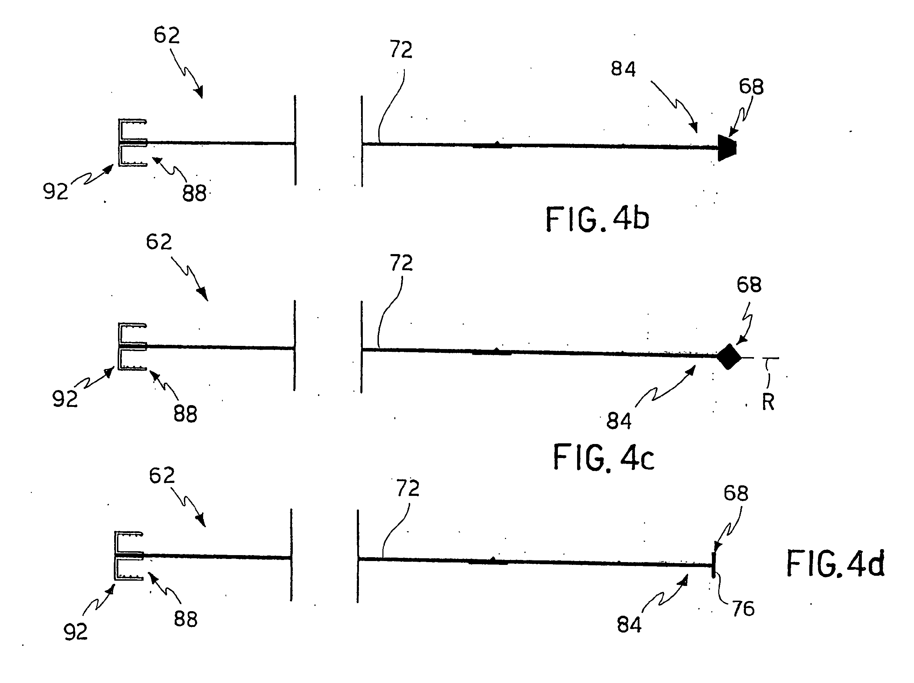

According to a further embodiment, shown in Figure 4B, the occluding body 68 is of frustoconical shape, having a major base and a minor base, such that, following the insertion of the occluding body 68 into the main cavity 20, the minor base faces towards the distal end 16 and the major base faces towards the proximal end 12.

According to a further embodiment, shown in Figure 4C, the occluding body 68 has a rhomboidal or lozenge shape, having a main axis of symmetry R which, in a configuration of mounting of the occluding body 68 in the catheter body 10, is disposed substantially parallel to the axis X.

According to a further embodiment, shown for example in Figure 4D, the occluding body 68 comprises a membrane 76 positioned at the distal end 16. The membrane 76 is disc-shaped and is suitable for at least partially occluding the main cavity of the catheter body 10.

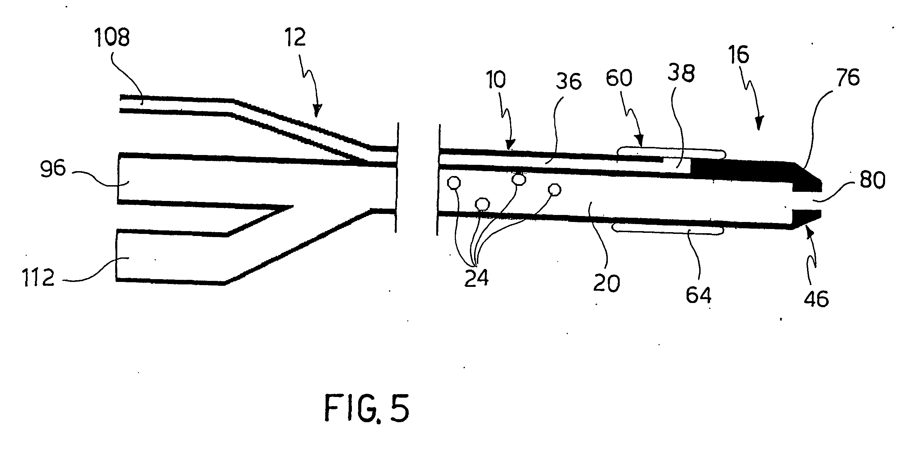

According to a further embodiment, shown in Figure 5, the membrane 76 is not connected to the insertion cable but is firmly fixed to the distal end 16 of the catheter body 10, so as to cap the main aperture 42. Preferably, the membrane 76 is disc-shaped, with a diameter substantially equal to the cavity of the distal end 16 and has a hole 80 suitable for allowing the passage of the guide cable of the catheter 4.

Preferably, this membrane 76 is of elastic material, so that the hole 80 which it has for the passage of the guide cable tends to close following the withdrawal of the cable itself.

The insertion cable 72 has a thickness less than the calibre of the main cavity 20 and, at one of its attachment ends 84, is connected to the occluding body 68.

Preferably, the insertion cable 72, at a fixing end 88 opposed to said attachment end 84, comprises threaded connection means 92, suitable for producing a threaded connection with a corresponding threaded portion of the catheter body 10 so as to effect the locking of the occluding body 68 within the main cavity 20.

Preferably, the insertion cable 72 has a length such that, following the insertion of the second occluding means 62 into the main cavity 20 and the relevant fixing to the catheter body 10, the occluding body 68 is positioned at a point comprised between the openings 24 and the distal end 16.

At the proximal end 12, the catheter body 10, as illustrated for example in Figure 3, comprises a main pathway 96 fluidly connected to the main cavity 20. Preferably, the main pathway 96 is coaxial with the main cavity 20 and has an internal calibre not less than the internal calibre of the main cavity 20.

By internal calibre of a cavity, there is to be understood the inside diameter of the cavity having a substantially cylindrical shape.

The main pathway 96 is suitable for receiving within it the guide cable for the insertion of the catheter, not shown.

The main pathway 96 is also suitable for receiving an occluding body to allow its introduction and positioning within the main cavity 20.

Preferably, the main pathway 96 comprises a threaded section 100 at a free end 104 thereof, said threaded section 100 being capable of engaging with the threaded connection means 92.of the second occluding means 62.

At the proximal end 12 the catheter body comprises a secondary pathway 108, hermetically separated from the main pathway 96 and fluidly connected to the secondary cavity 36, the secondary pathway 108 being suitable for receiving a fluid at the inlet and conveying it by means of the secondary cavity 36 to the first occluding means 60 to allow the actuation thereof.

For example, the secondary pathway 108 is suitable for being connected to a system for controlling the introduction of a fluid, comprising for example a tap (not shown), so as to be able to control and measure out the quantity of fluid introduced into the secondary cavity 36 and therefore the actuation of the first occluding means 60.

The catheter body 10, at the proximal end 12, further comprises an infusion pathway 112 which opens into the main cavity 20 and is suitable for receiving at the inlet a substance to be perfused and for directing it into the main cavity 20.

Preferably, the infusion pathway 112 comprises a threaded portion capable of being connected to means suitable for the controlled release of a substance, such as, for example, a contrast liquid.

A description will now be given of the technique to be used for perfusing a substance in a vessel through the catheter described above.

The technique consists in making a percutaneous puncture in a main or first vessel 6, for example a femoral artery, with a metal needle of dimensions such as to permit the passage of the guide cable for the catheter 4.

The guide cable is then introduced, bringing it at least as far as the branching or bifurcation 8 from which starts the secondary vessel 7 in which it is intended to perfuse a liquid, and the needle is withdrawn.

On the guide cable there is positioned a catheter introducer, typically for angiography catheters, using suitable dilators of increasing dimensions, and the catheter 4 is introduced and is pushed far enough to pass beyond, for example by three or four centimetres, the start of the second vessel 7 to be injected.

The contrast fluid is then injected as a preliminary, in order to evaluate the calibre of the main vessel, so as to establish the magnitude of the following inflation of the inflatable element 64, and the guide cable is withdrawn completely.

The occluding body 68 is introduced, effecting the positioning thereof for example by means of screwing home the threaded connection means 92 onto the corresponding main pathway 96.

Advantageously, the positioning of the occluding body 68 is effected in a portion comprised between the openings 24 and the distal end 16, so as to occlude or sub-occlude the main cavity 20.

The inflatable element 64 is then inflated, by injecting a predetermined quantity of liquid into the secondary cavity to inflate the inflatable element 64 such that the latter comes into contact with the inner wall 32 of the first vessel 7 and it occludes or sub-occludes the gap 63.

It is then possible to inject the substance to be perfused, for example a contrast liquid, through the infusion pathway 112 which opens into the main cavity 20. The liquid which flows from the proximal end 12 to the distal end 16 encounters a resistance to its passage, owing to the first and second occluding means which substantially limit any blow-by beyond the distal end through the main aperture 42; the liquid injected then flows down through the plurality of openings 24 suitably disposed at the bifurcation 8 from which starts the second vessel 7 to be perfused.

As can be understood from what has been described, the catheter according to the invention makes it possible to overcome the drawbacks exhibited by the catheters of the prior art.

Advantageously, the catheter described makes it possible also to select vessels of small calibre, avoiding cannulating them directly and therefore without running the risk of damaging them, for example dissecting them.

The catheter described therefore makes it possible to carry out investigations on blood vessels in a substantially non-traumatic manner for the latter, in so far as direct cannulation thereof is avoided.

For example, such a solution makes it possible to select the mammary artery indirectly from its point of origin from the subclavian artery. This is useful preoperatively, since it makes it possible to verify the quality of the mammary arteries and the feasibility of a "Y" anastomosis intervention between them; and postoperatively in the case of patients already operated on with interventions based on the use of both the mammary arteries anastomosed with one another. If all or the major part of the by-passes in a patient receive a flow, for example from the mammary artery sx, a lesion at this level could result in very serious ischemia. Therefore, since a conventional direct infusion catheter could cause serious damage to the vessel, normally in such patients post-operative checks are not carried out unless there are serious signs of post-operative ischemia (also for medico-legal reasons).

By means of the catheter described, a non-invasive diagnostic investigation can be carried out with contrast fluid, even on the mammary artery, which may advantageously be anastomosed for example with coronary vessels or with sections of saphenous vein.

Advantageously, the catheter described may be used selectively and non-traumatically for infusing any type of substance in the blood vessels, without the risk of causing lesions thereto and without producing useless dispersions of the substance injected.

In fact, the catheter described makes it possible to channel a flow of substance directly into the preselected vessel, avoiding dispersing same into other branchings.

The occluding body is further advantageously made of a material suitable for being subjected to sterilization treatment, for example a metallic or ceramic material, so that it can be sterilized according to known methods and re-used several times with different catheters.

Moreover, the combined occluding effect of the first and second occluding means facilitates the operation of occlusion of the distal end of the catheter. In particular, by inflating the balloon, the effect obtained is also a partial restriction of the calibre of the main cavity of the catheter, so that it is possible to use occluding bodies having dimensions smaller than said calibre. In this way the insertion of the occluding body into the cavity is facilitated, avoiding the risk of the occurrence of any jamming thereof in the phase of insertion into the main cavity.

The tapered profile, for example frustoconical, of the distal end, since it restricts the calibre of the main aperture, makes it possible to use occluding bodies of dimensions smaller than the median calibre of the main cavity and therefore does not require particularly narrow tolerances for the calibre itself. The substantially hermetic closure of the main cavity is ensured by the contact surface between the occluding body and the distal end which is advantageously a limited contact surface, for example, circumferential for a spherical occluding body or frustoconical for a frustoconical or rhomboidal occluding body. Moreover, owing to the tapered profile, an end of travel of the occluding body is guaranteed and a relatively high specific contact pressure between the occluding body and the distal end, so as to guarantee the substantially hermetic closure of the distal end.

Advantageously, the costs of production of the catheter are significantly reduced in so far as, since a clearance is allowed between the occluding body and the main cavity, particularly narrow tolerances are not required on the calibre of the main cavity.

An expert in the field, for the purpose of fulfilling contingent and specific requirements, may apply numerous modifications and variants to the catheter described above, all however contained within the scope of the invention as defined by the following claims.

## Claims

1. A catheter (4) for medical applications, suitable for being inserted into a duct (5) comprising a first vessel (6) and a second vessel (7) which branches off from said first vessel (6), the catheter (4) comprising a catheter body (10) which extends from a proximal end (12) to a distal end (16), said catheter body (10) comprising a main cavity (20), bounded by a lateral wall (28), which passes through the catheter body (10) between the proximal end (12) and the distal end (16), the main cavity (20) opening into a main aperture (42) at said distal end (16) and being suitable for receiving a guide cable for the insertion of the catheter (4) into the first vessel (6), and a plurality of openings (24), disposed on the lateral wall (28) at the distal end (16) and suitable for perfusing a substance, the catheter body (10), at a portion of the lateral wall (28) comprised between said plurality of openings (24) and said distal end (16), comprises first and second occluding means (60, 62), wherein the first occluding means (60) are suitable for at least partially occluding a gap (63) between the catheter body (10) and an inner wall (32) of the first vessel (6), and the second occluding means (62) can be associated internally with said main cavity (20) and are suitable for at least partially occluding said main cavity (20), said first and second occluding means (60, 62) defining a preferred direction of outflow of a fluid from the main cavity (20) of the catheter body (10) to the second vessel (7), through said plurality of openings (24) of the catheter body (10);
**characterised in that**
all the openings of said plurality of openings (24) pass through said lateral wall (28) and are in fluid communication with the main cavity (20)
said plurality of openings (24) are of such dimensions that the sum of the areas of the openings (24) is not less than the area of the main aperture (42) of the main cavity (20) of the catheter body (10) at the distal end (16)
said plurality of openings (24) are not aligned with one another with respect to a main axis of extension (X) of the catheter body (10).

2. A catheter according to claim 1, wherein said openings (24) are disposed substantially in a helical direction with respect to the main axis of extension (X) of the catheter body (10).

3. A catheter according to claim 1 or 2, wherein said first and second occluding means (60, 62) co-operate with each other to create a resistance to the passage of fluid through said distal end (16), favouring an outflow of fluid through said at least one opening (24).

4. A catheter according to claim 1, 2 or 3, wherein said first and second occluding means (60, 62), at a portion of the catheter body (10) comprised between said at least one opening (24) and said distal end (16), substantially effect the occlusion of the first vessel (6) into which the catheter (4) is inserted, so as to direct a flow of fluid into the second vessel (7), through said at least one opening (24).

5. A catheter according to any one of the preceding claims, wherein said first occluding means (60) comprise an inflatable element (64) positioned round the catheter body (10), said inflatable element (64), in a rest state, adhering substantially to the catheter body (10), and in a working state being substantially in contact with an inner wall (32) of said first vessel (6).

6. A catheter according to claim 5, wherein said inflatable element (64) is in fluid connection with the proximal end (12) so as to be operable from said proximal end (12).

7. A catheter according to any one of the preceding claims, wherein said catheter body (10) comprises a secondary cavity (36), which extends from the proximal end (12) to the distal end (16) and is hermetically separated from said main cavity (20), said secondary cavity (36) being in fluid connection with said first occluding means (60) so as to permit the actuation of said first occluding means (60).

8. A catheter according to claim 7, wherein said secondary cavity (36) is produced in a thickness of said lateral wall (28) of said catheter body (10).

9. A catheter according to claim 7 or 8, wherein the catheter body (10) has an oval cross-section having a first pole (37') more pronounced than a second pole (37") diametrically opposed to the first pole (37'), so that the lateral wall (28), at the first pole (37'), receives said secondary cavity (36).

10. A catheter according to any one of the preceding claims, wherein said second occluding means (62) comprise an occluding body (68), suitable for being introduced into said main cavity (20), and an insertion cable (72) firmly connected to said occluding body (68) so as to allow the insertion of the occluding body (68) through the main cavity (20).

11. A catheter according to claim 10, wherein said occluding body (68) is substantially spherical in shape.

12. A catheter according to claim 10, wherein said occluding body (68) is substantially frustoconical in shape.

13. A catheter according to any one of the preceding claims, wherein said catheter body (10), at said distal end (16), comprises a portion with tapered profile (46) so as to reduce the cavity of the catheter body (10) at the distal end (16).

14. A catheter according to any one of the preceding claims, wherein said second occluding means (62), at said distal end (16), comprise a membrane (76) suitable for at least partially occluding said main cavity (20) and having a hole (80) suitable for allowing the passage of the guide cable of said catheter (4).

15. A catheter according to claim 14, wherein said membrane (76) is firmly connected to the distal end (16) of the catheter body (10).

16. A catheter according to any one of the preceding claims, wherein said second occluding means (62) are made of a material suitable for being sterilized.

17. A catheter according to any one of the preceding claims, comprising, at said proximal end (12), a main pathway (96), suitable for receiving said second occluding means (62) and fluidly connected to said main cavity (20).

18. A catheter according to claim 17, wherein said main pathway (96) comprises a threaded section (100) capable of producing a threaded connection with a corresponding threaded portion of said second occluding means (62).

19. A catheter according to any one of the preceding claims, wherein said proximal end (12) comprises a secondary pathway (108), fluidly connected to said secondary cavity (36), and suitable for receiving at the inlet a fluid for allowing the actuation of said first occluding means (60).

20. A catheter according to any one of the preceding claims, wherein said proximal end (12) comprises an infusion pathway (112), fluidly connected to said main cavity (20) and suitable for receiving at the inlet a fluid, so as to allow the flow of the fluid from the proximal end (12) to the distal end (16).

## Patentansprüche

1. Katheter (4) für medizinische Anwendungen, geeignet zum Einführen in einen Gefäßgang (5), der ein erstes Gefäß (6) und ein zweites Gefäß (7) umfasst, das von dem ersten Gefäß (6) abzweigt, wobei der Katheter (4) einen Katheterkörper (10) aufweist, der sich von einem körpernahen Ende (12) zu einem körperfernen Ende (16) erstreckt und einen Haupthohlraum (20), begrenzt von einer Seitenwand (28), aufweist, die durch den Katheterkörper (10) zwischen dem körpernahen Ende (12) und dem körperfernen Ende (16) verläuft, der Haupthohlraum (20) zu einer Hauptöffnung (42) an dem körperfernen Ende (16) sich öffnet und dafür geeignet ist, ein Führungskabel zum Einschieben des Katheters (4) in das erste Gefäß (6) aufzunehmen und mit einer Vielzahl von Öffnungen (24) in der Seitenwand (28) am körperfernen Ende (16), durch die eine Substanz hindurchströmen kann, der Katheterkörper (10) in einem Abschnitt der Seitenwand (28) zwischen der Vielzahl von Öffnungen (24) und dem körperfernen Ende (16) erste und zweite abschließende Mittel (60, 62) aufweist, wobei die ersten abschließenden Mittel (60) zumindest teilweise einen Spalt (63) zwischen dem Katheterkörper (10) und einer Innenwand (32) des ersten Gefäßes (6) abschließen und die zweiten abschließenden Mittel (62) intern dem Haupthohlraum (20) zugeordnet sind und zumindest teilweise den Haupthohlraum (20) abschließen, die ersten und zweiten abschließenden Mittel (60, 62) eine bevorzugte Ausflussrichtung eines Fluids von dem Haupthohlraum (20) des Katheterkörpers (10) zu dem zweiten Gefäß (7) durch die Vielzahl von Öffnungen (24) des Katheterkörpers (10) festlegen, **dadurch gekennzeichnet, dass** alle Öffnungen der Vielzahl von Öffnungen (24) die Seitenwand (28) durchsetzen und in Fluidverbindung mit dem Haupthohlraum (20) stehen, dass die Vielzahl von Öffnungen (24) derartige Abmessungen hat, dass die Summe der Öffnungsflächen zumindest gleich der Fläche der Hauptöffnung (42) des Haupthohlraumes (20) des Katheterkörpers (10) am körperfernen Ende (16) ist, und dass die Vielzahl von Öffnungen (24) untereinander in Bezug auf eine Hauptachse (X) der Längsausdehnung des Katheterkörpers (10) nicht ausgerichtet ist.

2. Katheter nach Anspruch 1, **dadurch gekennzeichnet, dass** die Öffnungen (24) im wesentlichen wendelförmig in Bezug auf die Hauptachse (X) der Längsausdehnung des Katheterkörpers (10) angeordnet sind.

3. Katheter nach Anspruch 1 oder 2, **dadurch gekennzeichnet, dass** die ersten und zweiten abschließenden Mittel (60, 62) miteinander zusammenwirken, um einen Widerstand gegen den Fluiddurchfluss durch das körperferne Ende (16) zu schaffen, um einen Ausfluss des Fluids durch zumindest eine Öffnung (24) zu unterstützen.

4. Katheter nach Anspruch 1, 2 oder 3, **dadurch gekennzeichnet, dass** die ersten und zweiten abschließenden Mittel (60, 62) in einem Abschnitt des Katheterkörpers (10) zwischen mindestens einer Öffnung (24) und dem körperfernen Ende (16) im wesentlichen das Verschließen des ersten Gefäßes (6) bewirken, in das der Katheter (4) eingeschoben ist, um so einen Fluss des Fluids in das zweite Gefäß (7) durch zumindest die eine Öffnung (24) zu lenken.

5. Katheter nach jedem der voranstehenden Ansprüche, **dadurch gekennzeichnet, dass** die ersten abschließenden Mittel (60) ein aufblasbares Element (64) umfassen, das rings um den Katheterkörper (10) positioniert ist, wobei das aufblasbare Element (64) in einem Ruhezustand an dem Katheterkörper (10) anliegt und in einem Arbeitszustand im wesentlichen in Kontakt mit einer Innenwand (32) des ersten Gefäßes (6) sich befindet.

6. Katheter nach Anspruch 5, **dadurch gekennzeichnet, dass** das aufblasbare Element (64) in Fluidverbindung mit dem körpernahen Ende (12) steht, so dass es von diesem körpernahen Ende (12) aus betätigbar ist.

7. Katheter nach jedem der voranstehenden Ansprüche, **dadurch gekennzeichnet, dass** der Katheterkörper (10) einen Sekundärhohlraum (36) umfasst, der sich von dem körpernahen Ende (12) zu dem körperfernen Ende (16) erstreckt und hermetisch von dem Haupthohlraum (20) getrennt ist, und dass der Sekundärhohlraum (36) in Fluidverbindung mit den ersten abschließenden Mitteln (60) steht, um so die Betätigung der ersten abschließenden Mittel (60) zuzulassen.

8. Katheter nach Anspruch 7, **dadurch gekennzeichnet, dass** der Sekundärhohlraum (36) in einer Dicke gleich derjenigen der Seitenwand (28) des Katheterkörpers (10) gefertigt ist.

9. Katheter nach Anspruch 7 oder 8, **dadurch gekennzeichnet, dass** der Katheterkörper (10) einen ovalen Querschnitt aufweist, der einen ersten Pol (37') hat, der deutlicher hervorsteht als ein zweiter Pol (37"), diametral gegenüber liegend dem ersten Pol (37'), so dass die Seitenwand (28) am ersten Pol (37') den zweiten Sekundärhohlraum (36) aufnehmen kann.

10. Katheter nach jedem der voranstehenden Ansprüche, **dadurch gekennzeichnet, dass** die zweiten abschließenden Mittel (32) einen Abschlusskörper (68) umfassen, der in den Haupthohlraum (20) einschiebbar ist und ein Einschubkabel (72), das mit dem Abschlusskörper (68) fest verbunden ist, um so den Einschub des Abschlusskörpers (68) durch den Haupthohlraum (20) zu ermöglichen.

11. Katheter nach Anspruch 10, **dadurch gekennzeichnet, dass** der Abschlusskörper (68) im wesentlichen sphärische Gestalt hat.

12. Katheter nach Anspruch 10, **dadurch gekennzeichnet, dass** der Abschlusskörper (68) im wesentlichen abgestumpfte Kegelgestalt hat.

13. Katheter nach jedem der voranstehenden Ansprüche, **dadurch gekennzeichnet, dass** der Katheterkörper (10) am körperfernen Ende einen Abschnitt mit schräg zusammenlaufendem Profil (46) aufweist, um so den Hohlraum des Katheterkörpers (10) am körperfernen Ende (16) zu reduzieren.

14. Katheter nach jedem der voranstehenden Ansprüche, **dadurch gekennzeichnet, dass** die zweiten abschließenden Mittel (62) am körperfernen Ende (16) eine Membran (76) umfassen, die zumindest teilweise den Haupthohlraum (20) abschließt und ein Loch (80) hat, durch das das Führungskabel des Katheters (4) hindurchgeführt ist.

15. Katheter nach Anspruch 14, **dadurch gekennzeichnet, dass** die Membran (76) fest mit dem körperfernen Ende (16) des Katheterkörpers (10) verbunden ist.

16. Katheter nach jedem der voranstehenden Ansprüche, **dadurch gekennzeichnet, dass** das Material der zweiten abschließenden Mittel (62) sterilisierbar ist.

17. Katheter nach jedem der voranstehenden Ansprüche umfassend am körpernahen Ende einen Hauptpfad (96), der die zweiten abschließenden Mittel (62) aufnimmt und fluidmäßig mit dem Haupthohlraum (20) verbunden ist.

18. Katheter nach Anspruch 17, **dadurch gekennzeichnet, dass** der Hauptpfad (96) einen Gewindeabschnitt (100) aufweist, zur Herstellung einer Schraubverbindung mit einem entsprechenden Gewindeabschnitt der zweiten abschließende Mittel (62).

19. Katheter nach jedem der voranstehenden Ansprüche, **dadurch gekennzeichnet, dass** das körpernahe Ende (12) einen Sekundärpfad (108) aufweist, der fluidmäßig mit dem Sekundärhohlraum (36) verbunden ist und an dem Einlass ein Fluid empfängt, das die Betätigung der ersten abschließenden Mittel (60) ermöglicht.

20. Katheter nach jedem der voranstehenden Ansprüche, **dadurch gekennzeichnet, dass** das körpenahe Ende (12) einen Infusionspfad (112) enthält, der fluidmäßig mit dem Haupthohlraum (20) verbunden ist und am Einlass ein Fluid empfängt, das den Fluidfluss von dem körpernahen Ende (12) zu dem körperfernen Ende (16) ermöglicht.

## Revendications

1. Cathéter (4) pour applications médicales, convenant pour être inséré dans un conduit (5) comprenant un premier vaisseau (6) et un deuxième vaisseau (7) qui est dérivé du dit premier vaisseau (6), le cathéter (4) comprenant un corps de cathéter (10) qui s'étend d'une extrémité proximale (12) à une extrémité distale (16), le dit corps de cathéter (10) comprenant une cavité principale (20), délimitée par une paroi latérale (28), qui passe dans le corps de cathéter (10) entre l'extrémité proximale (12) et l'extrémité distale (16), la cavité principale (20) débouchant dans un orifice principal (42) à la dite extrémité distale (16) et convenant pour recevoir un câble de guidage pour l'insertion du cathéter (4) dans le premier vaisseau (6), et une pluralité de trous (24) ménagés dans la paroi latérale (28) à l'extrémité distale (16) et convenant pour la perfusion d'une substance,
le corps de cathéter (10) dans une partie de la paroi latérale (28) comprise entre la dite pluralité de trous (24) et la dite extrémité distale (16), comprend un premier et un deuxième moyens d'occlusion (60, 62), le premier moyen d'occlusion (60) convenant pour obturer au moins en partie un intervalle (63) entre le corps de cathéter (10) et une paroi intérieure (32) du premier vaisseau (6), et le deuxième moyen d'occlusion (62) peut être associé intérieurement avec la dite cavité principale (20) et il convient pour obturer au moins partiellement la dite cavité principale (20),
les dits premier et deuxième moyens d'occlusion (60, 62) définissant une direction préférée de circulation d'un fluide à partir de la cavité principale (20) du corps de cathéter (10) vers le deuxième vaisseau (7), à travers la dite pluralité de trous (24) du corps de cathéter (10) ;
**caractérisé en ce que** :
tous les trous de la dite pluralité de trous (24) traversent la dite paroi latérale (28) et sont en communication de fluide avec la cavité principale (20),
la dite pluralité de trous (24) ont des dimensions telles que la somme des surfaces des trous (24) n'est pas inférieure à la surface de l'orifice principal (42) de la cavité principale (20) du corps de cathéter (10) à l'extrémité distale (16), et
la dite pluralité de trous (24) ne sont pas alignés les uns avec les autres par rapport à un axe principal d'extension (X) du corps de cathéter (10).

2. Cathéter selon la revendication 1, dans lequel les dits trous (24) sont disposés sensiblement dans une direction hélicoïdale par rapport à l'axe principal d'extension (X) du corps de cathéter (10).

3. Cathéter selon la revendication 1 ou 2, dans lequel les dits premier et deuxième moyens d'occlusion (60, 62) coopèrent ensemble pour créer une résistance au passage de fluide à travers la dite extrémité distale (16), favorisant une sortie de fluide à travers le dit au moins un trou (24).

4. Cathéter selon la revendication 1, 2 ou 3, dans lequel les dits premier et deuxième moyens d'occlusion (60, 62), dans une partie du corps de cathéter (10) comprise entre le dit au moins un trou (24) et la dite extrémité distale (16), effectuent sensiblement l'occlusion du premier vaisseau (6) dans lequel le cathéter (4) est inséré, de façon à diriger un flux de fluide dans le deuxième vaisseau (7), à travers le dit au moins un trou (24).

5. Cathéter selon une quelconque des revendications précédentes, dans lequel le dit premier moyen d'occlusion (60) comprend un élément gonflable (64) placé autour du corps de cathéter (10), le dit élément gonflable (64), dans un état de repos, adhérant substantiellement au corps de cathéter (10) et, dans un état de travail, étant sensiblement en contact avec une paroi intérieure (32) du dit premier vaisseau (6).

6. Cathéter selon la revendication 5, dans lequel le dit élément gonflable (64) est un communication de fluide avec l'extrémité proximale (12) de façon à être actionnable à partir de la dite extrémité proximale (12).

7. Cathéter selon une quelconque des revendications précédentes, dans lequel le dit corps de cathéter (10) comprend une cavité secondaire (36) qui s'étend de l'extrémité proximale (12) à l'extrémité distale (16) et est hermétiquement séparée de la dite cavité principale (20), la dite cavité secondaire (36) étant en connexion de fluide avec le dit premier moyen d'occlusion (60) de façon à permettre la commande du dit premier moyen d'occlusion (60).

8. Cathéter selon la revendication 7, dans lequel la dite cavité secondaire (36) est créée dans une épaisseur de la dite paroi latérale (28) du dit corps de cathéter (10).

9. Cathéter selon la revendication 7 ou 8, dans lequel le corps de cathéter (10) a une section transversale ovale ayant un premier pôle (37') plus prononcé qu'un deuxième pôle (37") diamétralement opposé au premier pôle (37'), de sorte que la paroi latérale (28), au premier pôle (37'), reçoit la dite cavité secondaire (36).

10. Cathéter selon une quelconque des revendications précédentes, dans lequel le dit deuxième moyen d'occlusion (62) comprend un élément d'occlusion (68), convenant pour être introduit dans la dite cavité principale (20), et un câble d'insertion (72) solidement relié au dit élément d'occlusion (68) de façon à permettre l'insertion de l'élément d'occlusion (68) à travers la cavité principale (20).

11. Cathéter selon la revendication 10, dans lequel le dit élément d'occlusion (68) est de forme sensiblement sphérique.

12. Cathéter selon la revendication 10, dans lequel le dit élément d'occlusion (68) est de forme sensiblement tronconique.

13. Cathéter selon une quelconque des revendications précédentes, dans lequel le dit corps de cathéter (10), à la dite extrémité distale (16), comprend une portion de profil conique (46) de façon à réduire la cavité du corps de cathéter (10) à l'extrémité distale (16).

14. Cathéter selon une quelconque des revendications précédentes, dans lequel le dit deuxième moyen d'occlusion (62), à la dite extrémité distale (16), comprend une membrane (76) convenant pour obturer au moins partiellement la dite cavité principale (20) et ayant un trou (80) approprié pour permettre le passage du câble de guidage du dit cathéter (4).

15. Cathéter selon la revendication 14, dans lequel la dite membrane (76) est solidement reliée à l'extrémité distale (16) du corps de cathéter (10).

16. Cathéter selon une quelconque des revendications précédentes, dans lequel le dit deuxième moyen d'occlusion (62) est fabriqué en une matière pouvant être stérilisée.

17. Cathéter selon une quelconque des revendications précédentes, comprenant, à la dite extrémité proximale (12), un passage principal (96) convenant pour recevoir le dit deuxième moyen d'occlusion (62) et en communication de fluide avec la dite cavité principale (20).

18. Cathéter selon la revendication 17, dans lequel le dit passage principal (96) comprend une partie filetée (100) permettant d'effectuer une connexion vissée avec une partie filetée correspondante du dit deuxième moyen d'occlusion (62).

19. Cathéter selon une quelconque des revendications précédentes, dans lequel la dite extrémité proximale (12) comprend un passage secondaire (108) en connexion de fluide avec la dite deuxième cavité secondaire (36) et convenant pour recevoir à l'entrée un fluide qui permet de commander le dit premier moyen d'occlusion (60).

20. Cathéter selon une quelconque des revendications précédentes, dans lequel la dite extrémité proximale (12) comprend un passage de perfusion (112), en connexion de fluide avec la dite cavité principale (20) et prévu pour recevoir à l'entrée un fluide, afin de permettre l'écoulement du fluide de l'extrémité proximale (12) à l'extrémité distale (16).
